# EUROPEAN PATENT SPECIFICATION

(11) **EP 2 389 974 B1**
(45) Date of publication and mention of the grant of the patent: **23.04.2014**
(21) Application number: 10450091.3
(22) Date of filing: 25.05.2010
(51) Int. Cl.: A61M 25/10, A61M 1/10, A61M 25/00

(54) **A balloon catheter for introduction into a body vessel, in particular the coronary sinus**
Ballonkatheter zur Einführung in ein Körpergefäß, insbesondere in den Koronarsinus
Cathéter à ballonnet pour introduction dans un vaisseau corporel, en particulier le sinus coronaire

(43) Date of publication of application: 30.11.2011
(73) Proprietor: Miracor Medical Systems GmbH, 1060 Vienna (AT)
(72) Inventor: Kohr, Oliver A., CH-6314 Unteraegeri (CH); Hoem, Jon H., CH-6315 Oberaegeri (CH)
(74) Representative: Keschmann, Marc

(56) References cited:
- EP-A1- 1 611 914
- WO-A1-03/041783
- US-A- 3 995 623
- US-A- 4 931 036
- US-A- 5 242 396
- US-A- 5 324 260
- US-A- 5 456 665
- US-A- 5 507 766
- US-A- 5 683 347
- US-A1- 2003 032 974
- US-A1- 2004 172 004
- US-B1- 6 179 856
- US-B1- 6 500 145
- US-B1- 6 673 040

## Description

The invention relates to a balloon catheter for introduction into a body vessel, in particular the coronary sinus, and the occlusion of the same, comprising a catheter shaft that carries an inflatable balloon on a distal end portion and a plurality of lumens extending through the catheter shaft.

Balloon catheters including inflatable balloons can, for instance, be taken from EP 402964 B1. The known balloon catheter serves for coronary sinus occlusion, wherein diagnostically valuable signals can be obtained by a plurality of sensors and the inflation of the balloon can be controlled, in particular, with a view to achieving retroperfusion. Such a balloon catheter is also known as a multi-lumen catheter, whose distal end projects, for instance, into the coronary sinus of the heart, while the proximal end of the catheter is connected with a pump for inflating the balloon. Wires for electrically contacting sensors can be conducted through further lumens arranged coaxially or in parallel with the lumen that serves to inflate the expandable balloon. Via such further lumens, cardioplegic or thrombolytic, or other pharmacologically active substances suitable for retroperfusion in ischemic tissue, can also be introduced.

In order to supply ischemic tissue with blood by retrograde perfusion, it has already been proposed to use an inflatable balloon fixed to the end of a catheter to intermittently occlude the coronary sinus. The blood pressure in the coronary sinus rises during the occlusion at every heart beat so as to cause blood reaching the coronary sinus through the healthy tissue of the heart muscle to be flushed back into the ischemic tissue. Another effect of intermittent occlusion is the influence on the pressure regulation due to the feedback mechanism by neurostimulative effects. For such intermittent coronary sinus occlusion, the balloon end of the catheter is inserted either percutaneously or surgically. The other end of the catheter is supplied by a pump with a gas or fluid which causes the cyclic inflation and deflation of the balloon. A device for the retroperfusion of coronary veins is, for instance, known from WO 2005/120602 A1, by which a pressure-controlled, intermittent coronary sinus occlusion can be performed. In that device and the corresponding method for determining the optimum times for triggering and releasing the occlusion, pressure parameters like the speeds of the pressure increase and pressure drop were determined and subjected to relatively complex processing.

For the percutaneous insertion of a catheter, it is proceeded in a manner that the catheter is guided via the inferior or the superior caval vein into the right atrium of the heart, into which the coronary sinus runs. Due to the position of the mouth of the superior caval vein or inferior caval vein, respectively, relative to the mouth of the coronary sinus, the introduction of the catheter into the coronary sinus requires considerable skill from the surgeon in order to direct the tip of the catheter, or a guide wire or a guide sleeve, into the coronary sinus in such a manner as to enable the subsequent introduction of the catheter along with the occlusion device. In fact, it frequently happened that several attempts of introduction into the coronary sinus had to be made, which considerably extended the duration of treatment and, hence, the strain on the patient.

Another problem involved in balloon catheters used for the intermittent occlusion of the coronary sinus resides in that blood backed up during the occlusion would exert pressure on the balloon, and hence on the catheter, thus eventually causing the catheter to slip back within the vessel. It is, therefore, necessary to configure the catheter sufficiently rigid in order to prevent the catheter from evading, e.g. laterally, in certain points because of the counter-pressure, which might cause the balloon to slip back. At the same time, sufficient flexibility of the catheter must be safeguarded in order to enable the catheter to be safely pushed forward through blood vessel regions having small radii of curvature so as to be able to position the balloon on the desired site of application and, in particular, in the coronary sinus.

Catheters of the initially mentioned kind being equipped with stiffening elements influencing the flexibility and/or rigidity of the catheter shaft are disclosed in EP 1611914 A1, US 5,683,347 A and US 5,456,665 A. EP 1611914 A1 discloses a hypotube, which is affixed to and surrounds a portion of the inner body of the catheter shaft. The hypotube has a cylindrical segment and a spirally cut segment. The spirally cut segment provides a graduated transmission in bending flexibility.

The invention, therefore, aims to provide a balloon catheter which is suitable for the intermittent occlusion of body vessels and, in particular, the coronary sinus, and which is equipped with the lumens required therefor, and which, at the same time, exhibits both sufficient flexural strength to enhance the pushability of the catheter, and ensure that the balloon will not slip back on account of the pressure caused by the backed-up blood, and sufficient flexibility to facilitate its introduction.

US 5507766 discloses a balloon catheter for introduction into a body vessel comprising a catheter shaft that carries an inflatable balloon. The dilatation instrument comprises an inner tube defining a first lumen and an outer tube disposed coaxially around the inner tube and defining a second lumen. One end of the inflatable member is attached to the inner tube and the other end to the outer tube. The catheter comprises a stiffening element which shows a distal end portion which is positioned adjacent to the proximal end of the balloon and has a flexural strength reduced relative to a remaining portion of the stiffening element.

US 5242396 describes a balloon catheter for introduction into a body vessel, in particular the coronary sinus, and for intermittently occluding the same, comprising a catheter shaft and carrying an inflatable balloon on a distal end portion and a plurality of lumens extending through the catheter shaft.

To solve this object, the balloon catheter of the initially defined kind exhibits a pressure sensor lumen extending centrally through the catheter shaft to at least one distal opening, the at least one opening being arranged distally of the balloon for communication with the fluid in the body vessel. Furthermore, an inflation lumen to inflate and deflate the balloon is provided, and the catheter comprises a stiffening element extending along the catheter shaft, the stiffening element having a distal end portion that is positioned adjacent to the proximal end of the balloon and has a flexural strength that is reduced relative to a remaining portion of the stiffening element. Also, a balloon pressure-monitoring lumen to measure the pressure in the balloon is further provided. Due to the fact that a central lumen is provided, whose distal opening is in communication with the fluid in the body vessel distally of the balloon, it is, for instance, possible to measure, via said lumen, the pressure prevailing in the body vessel, in particular the coronary sinus, occluded by the balloon. Said central lumen also enables the taking of blood from the occluded vessel, in particular coronary sinus. Moreover, the central lumen can be used to introduce the balloon catheter into the vessel, and advance it within the vessel to the respectively targeted site, by advancing the central lumen over a guide wire.

The flexural strength of the balloon catheter is obtained by the stiffening element provided according to the invention which according to a preferred embodiment of the invention surrounds the catheter shaft or is arranged within the catheter shaft. The stiffening element also facilitates the advancement of the balloon catheter. In order not to injure the vessel during the advancement of the catheter, and to ensure that an advancement will also be possible in curved regions having small radii of curvature, it is provided according to the invention that the distal end portion of the stiffening element, which is positioned adjacent to the proximal end of the balloon, has a flexural strength that is reduced relative to a remaining portion of the stiffening element. Thus, a region of higher flexibility is deliberately formed at the distal end portion of the stiffening element so as to enable the adaptation to a curved course of the body vessel during the advancement of the catheter, while constantly holding the balloon-carrying, distal end portion of the catheter shaft as parallel as possible with the longitudinal extension of the respective vessel in order to avoid injury to the vessel wall.

The stiffening element is preferably formed by a hypotube surrounding the catheter shaft. The stiffening element in this case preferably extends substantially over the entire portion of the catheter shaft between the balloon and a proximal hub. In a preferred manner, a slight distance is provided between the distal end of the stiffening element and the proximal end of the inflatable region of the balloon. Said distance must be dimensioned such that, on the one hand, the catheter shaft will not buckle between the distal end of the stiffening element and the balloon, which would be the case with too large a distance, and, on the other hand, the flexibility and suppleness will not be limited too much in this region, which would be the case with too short a distance, or no distance at all. The distance preferably is 4-6 mm and, in particular, about 5 mm.

The hypotube may be formed by a separate stainless-steel tube or also by at least one outer layer co-extruded with the catheter shaft and made of a synthetic material differing from that of the catheter shaft. Alternatively, the hypotube may be formed by a nylon tissue or comprise such a tissue.

It is preferably provided that the distal end portion having a reduced flexural strength extends over a length of 30-120 mm, preferably 40-90 mm.

From the prior art, various ways to make the flexural strength of a hypotube variable over its axial length have already become known. According to a preferred configuration, it is provided that the hypotube comprises at least one notch influencing the flexural strength. The notch preferably extends in a helical line-shaped manner, with the helical line or helix having a smaller pitch in the distal end portion of the stiffening element having the reduced flexural strength than in the remaining portion of the stiffening element. Further optimization is feasible in that the pitch of the helix continuously increases in the distal end portion of the stiffening element having the reduced flexural strength, departing from the distal end of the hypotube. Due to the continuously variable flexural strength of the hypotube in the mentioned end portion, buckling sites will be avoided.

Instead of a helical line-shaped notch, a plurality of notches offset in the axial direction may also be provided on the stiffening element, each extending over a partial circumference of the hypotube, with the flexural strength depending on the axial distance between the individual notches.

For the intermittent occlusion of the body vessel and, in particular, the coronary sinus, a plurality of lumens are usually required such that a particularly space-saving arrangement of the individual lumens will be useful in order to maintain the outer diameter of the catheter shaft as small as possible. The configuration in this respect is preferably further developed such that the lumens of the catheter have cross-sectional geometries differing from one another, the inflation lumen serving to inflate and/or deflate the balloon preferably being ring segment-shaped in cross section and arranged radially outside the central pressure sensor lumen.

In order enable the monitoring of the fluid pressure prevailing in the balloon, a separate lumen will be of advantage, and the configuration in this respect is preferably further developed such that a balloon pressure-monitoring lumen to measure the pressure in the balloon is further provided, which is ring segment-shaped in cross section and arranged radially outside the pressure sensor lumen, wherein the arc-determining angle of the balloon pressure-monitoring lumen is preferably smaller as compared to the ring segment-shaped inflation lumen. Due to the fact that the ring segment-shaped cross section of the inflation lumen extends over a larger central angle than the ring segment-shaped cross section of the balloon pressure-monitoring lumen, a cross section as large as possible will be provided for the inflation lumen and, at the same time, separate pressure measurements will be enabled, thus ensuring the optimum utilization of space while maintaining the outer diameter of the catheter shaft accordingly small.

Pressure measurement via a separate lumen, for instance, has the advantage that possible buckling under flexural load can be detected. Buckling will, in fact, be reliably recognized due to the different pressures measured in the inflation lumen and in the balloon pressure-monitoring lumen, and it will thus be feasible to take the respective safety measurements, e.g. actuate a safety valve, in due time.

According to a further preferred configuration, it is provided that a circular or oval lumen is provided between the neighbouring ends of the ring segment-shaped cross sections of the inflation lumen and the balloon pressure-monitoring lumen. Such a relatively small lumen enables the wiring of electrical sensors arranged on the tip of the catheter or in the region of the balloon, or the arrangement of fibre-optic lines.

In order to avoid possible malfunctions during the inflation and deflation of the balloon, it is provided according to a preferred further development that the inflation lumen, and optionally the balloon pressure-monitoring lumen, are each connected with the interior of the balloon via at least two radial openings arranged to be offset in the axial direction of the catheter shaft. If the inflating and deflating of the balloon merely took place via a single radial opening, this would involve the risk of the balloon prematurely covering this single opening, particularly during collapsing, i.e. prior to the complete evacuation of the balloon, so that further evacuation would be rendered difficult or impossible.

A further preferred configuration provides that the catheter comprises a stiffening means, in particular a stiffening wire, in the region of the balloon, said wire preferably extending over the length of the balloon. The stiffing means may also extend from a region interior to the previously describe hypotube to a distal region interior to a distal collar of the balloon. Such stiffening means and, in particular, stiffening wire can reduce the likelihood of the catheter shaft buckling or otherwise deforming in the region in which it is surrounded by the balloon, on account of the balloon pressure exerting axial upsetting forces on the catheter shaft. The stiffening means, in particular stiffening wire, can, for instance, be received in the circular or oval lumen mentioned above. In an alternative embodiment, the stiffening wire may be arranged in a helical wrap around an outer circumference of the catheter shaft in the interior region of the balloon.

In another preferred development the catheter comprises a catheter tip element in fluid connection with the pressure sensor lumen extending centrally through the catheter shaft, the catheter tip element comprising a plurality of distal ports that are positioned distally of the balloon for communication with fluid in the body vessel, the plurality of distal ports comprising a plurality of radial openings, preferably at least two, in particular three radial openings that are substantially uniformly distributed over a circumferential periphery of the catheter tip element. Such a separate catheter tip component offers various advantages in terms of application. Thus, a distal edge of a distal opening of the catheter tip element may, for instance, be rounded off so as to prevent damage to the vessel walls. Furthermore, the catheter tip element may conically taper toward the distal opening, and the catheter tip element may, in particular, be designed to be flexible in order to ensure an enhanced guidance of the tip, and hence the overall catheter, within the body vessel. In this respect, the catheter tip element should have a minimum length in order to provide an appropriate bending zone. In a preferred manner, the distance between the distal tip of the catheter tip element and the distal end of the inflatable portion of the balloon is at least 30 mm and, preferably, 35 to 45 mm.

By the axial lumen of the catheter tip element communicating with the body vessel not only via the distal opening of the catheter tip element, but also via at least two, preferably three, radial openings uniformly distributed over its periphery in the wall of the catheter tip element, a more precise measurement of the fluid pressure prevailing in the body vessel has become feasible. In particular, faulty measurements will be excluded, which would, for instance, occur if the distal opening or a radial opening were in abutment on a wall of the body vessel, or an opening were not powered with the full pressure prevailing in the vessel for any other reason.

In the following, the invention will be explained in more detail by way of an exemplary embodiment schematically illustrated in the drawing.
Fig. 1 is a perspective view of a portion of a system for treating heart tissue, in accordance with some embodiments.
Fig. 2 is a perspective view of another portion of the system of Fig. 1.
Fig. 3 is a schematic illustration of a catheter device of the system of Fig. 1.
Fig. 4 is a detailed view of the balloon of the catheter device of Fig. 3.
Fig. 5 shows a cross sectional view of the catheter device of Fig. 3.
Fig. 6 shows a cross sectional view of a portion of the catheter device of Fig. 4.
Fig. 7 is a cross-sectional view of a catheter tip element of the catheter device of Fig. 3.
Fig. 8 is a perspective view of the catheter tip element of Fig. 7.
Fig. 9 is a detailed view of a portion of the catheter of Fig. 3 with the balloon removed from the view.
Fig. 10 is a detail view of a stiffening element for the catheter of Fig. 3.

Like reference symbols in the various drawings indicate like elements.

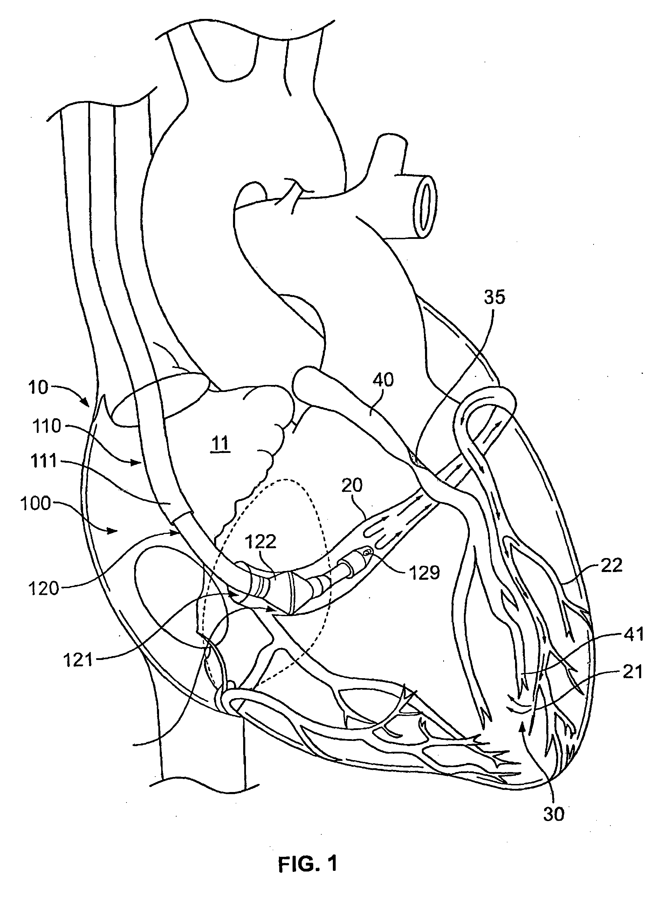

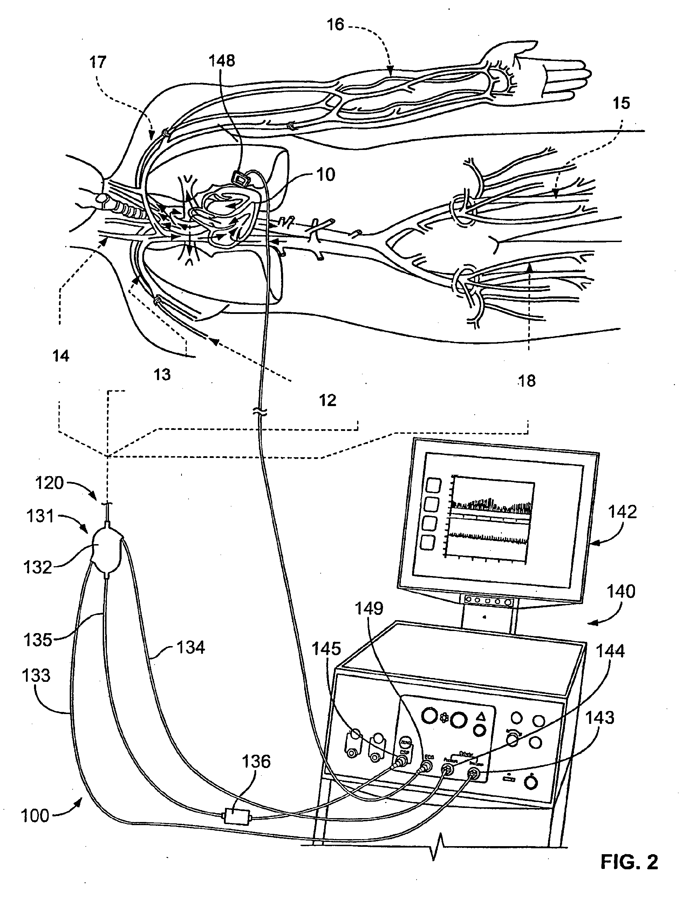

Referring to Figs. 1-2, some embodiments of a system 100 for treating heart tissue can include a coronary sinus occlusion catheter 120 and a control system 140 (Fig. 2). In particular embodiments, the control system 140 can be configured to control the operation of the catheter 120 for providing pressure-controlled intermittent coronary sinus occlusion (PICSO) and to receive heart sensor data for display. The coronary sinus occlusion catheter 120 includes a distal tip portion 121 (leading to a distal end depicted in Fig. 1) and a proximal portion 131, which includes a proximal hub 132 that is coupled to the control system 140 via a number of fluid or sensor lines 133, 134 and 135. Accordingly, the control system 140 may be employed to operate one or more components at the distal tip portion 121 of the coronary sinus occlusion catheter 120 while also receiving one or more sensor signals that provide data indicative of heart characteristics (e.g., coronary sinus pressure, electrocardiogram (ECG) information, and the like).

Briefly, in use, the distal tip portion 121 of the coronary sinus occlusion catheter 120 can be arranged in a coronary sinus 20 of a heart 10 and thereafter activated to intermittently occlude the blood flow exiting from the coronary sinus 20 and into the right atrium 11. During such an occlusion of the coronary sinus 20, the venous blood flow that is normally exiting from the coronary sinus 20 may be redistributed into a portion of heart muscle tissue 30 that has been damaged due to blood deprivation. For example, the portion of heart muscle tissue 30 can suffer from a lack of blood flow due to a blockage 35 in a coronary artery 40. As a result, the arterial blood flow to the affected heart muscle tissue 30 via a local artery 41 can be substantially reduced such that the heart muscle tissue 30 becomes ischemic or otherwise damaged. Further, because the arterial blood flow is reduced, the venous blood flow exiting from the local vein 21 is likewise reduced. Other branch veins 22 located at different regions along the heart 10 may continue to receive blood flow, thereby creating a supply of venous blood flow exiting through the coronary sinus 20. In some embodiments, the coronary sinus occlusion catheter 120 can be delivered into the coronary sinus 20 and thereafter activated so as to intermittently occlude the coronary sinus 20 before, during, or after treating the blockage 35 on the arterial side. Such an occlusion can cause the venous blood flow to be redistributed to the local vein 21 and then into the portion of heart muscle tissue 30 can suffer from a lack of blood flow due to a blockage 35 in a coronary artery 40. As such, the ischemic or otherwise damaged heart muscle tissue 30 can be treated with the redistributed venous blood flow in that the heart muscle tissue 30 receives redistribution of flow before, during, and after the blockage 35 is repaired or removed to restore normal coronary arterial blood flow.

Furthermore, in use, the control system 140 (Fig. 2) is configured to provide automated control of an occlusion component (e.g., an inflatable balloon 122 or the like) of the coronary sinus occlusion catheter 120. As described in more detail below, the control system 140 includes a computer processor that executes computer-readable instructions stored on a computer memory device so as to activate or deactivate the occlusion in the coronary sinus 20 in accordance with particular patterns. For instance, the control system 140 can be configured to activate the occlusion component of the catheter 120 in the coronary sinus 20 as part of a predetermined pattern of occlusion periods and release periods that is independent of the coronary sinus pressure, or as part of a pressure-dependent pattern that is at least partially defined by the coronary sinus pressure readings during the procedure. In addition, the control system 120 is equipped with a display device 142 having a graphical user interface that provides a cardiologist or other user with time-sensitive, relevant data indicative of the progress of a coronary sinus occlusion procedure and the condition of the heart 10. As such, the user can readily monitor the patient's condition and the effects of intermittently occluding the coronary sinus 20 by viewing the graphical user interface while contemporaneously handling the coronary sinus occlusion catheter 120 other heart treatment instruments (e.g., angioplasty catheters, stent delivery instruments, or the like). It should be understood from the description herein that, in some embodiments, the control system 140 and the coronary sinus occlusion catheter 120 can be used as part of a system for treating the heart muscle tissue before, during, and after the blockage 35 is repaired or removed to restore normal coronary arterial blood flow.

Referring in more detail to Fig. 1, the coronary sinus occlusion catheter 120 can be delivered via the venous system to the coronary sinus 20 before, during, or after repairing or treating the blockage 35 the coronary artery 40. In such circumstances, the portion of heart muscle tissue 30 that is damaged due to lack of arterial blood flow (as a result of the blockage) can be treated with a supply of venous blood while the normal arterial blood flow is restored (as a result of repairing or removing the blockage 35).

The system 100 may include a guide member 110 that is advanced through the venous system of the patient and into the right atrium 11. The guide member 110 in this embodiment comprises a guide sheath having a lumen extending between a distal end 111 (Fig. 1) and a proximal end. In alternative embodiments, the guide member 110 can include a guide wire having an exterior surface extending between the distal end and the proximal end. Optionally, the guide member 110 includes a steerable mechanism to control the orientation of the distal end so as to steer the distal end 111 through the venous system and into the right atrium 11. Also, the guide member 110 can include one or more marker bands along the distal end 111 so that the position of the distal end can be monitored during advancement using an imaging device.

After the guide member 110 is advanced into the right atrium 11, the distal end 111 may be temporarily positioned in the coronary sinus 20. From there, the distal tip portion 121 of the coronary sinus occlusion catheter 120 can be slidably advanced along the guide member 110 for positioning inside the coronary sinus 20. In the embodiments in which the guide member 110 comprises a guide sheath, the distal tip portion 121 of the coronary sinus occlusion catheter 120 can slidably engage with an interior surface of the lumen during advancement toward the coronary sinus 20. In the alternative embodiments in which the guide member 110 comprises a guide wire structure, the distal tip portion 121 of the coronary sinus occlusion catheter 120 can slidably advance over the exterior surface of the guide wire (e.g., a lumen of the catheter 120 passes over the guide wire) during advancement toward the coronary sinus 20. After the coronary sinus occlusion catheter 120 reaches the coronary sinus 20, the distal end 111 of the guide member 110 can be withdrawn from the coronary sinus 20 and remain in the right atrium 11 during use of the coronary sinus occlusion catheter 120.

Still referring to Fig. 1, the distal tip portion 121 of the coronary sinus occlusion catheter 120 that is positioned in the coronary sinus 20 includes an occlusion component 122, which in this embodiment is in the form of an inflatable balloon device. The occlusion component 122 can be activated so as to occlude the coronary sinus 20 and thereby cause redistribution of the venous blood into the heart muscle tissue 30 that is damaged due to a lack of arterial blood flow. As described in more detail below, the inflatable balloon device 122 can be in fluid communication with an internal lumen of the coronary sinus occlusion catheter 120, which is in turn in communication with a pneumatic subsystem of the control system 140 (Fig. 2). As such, the control system 140 can be employed to expand or deflate the balloon device 122 in the coronary sinus.

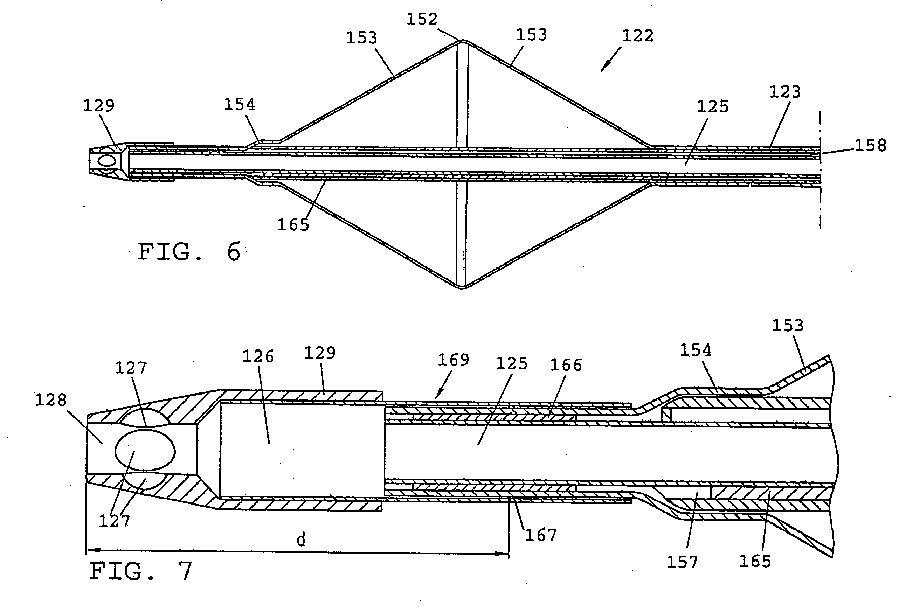

The distal tip portion 121 also includes a tip element 129 having one or more distal ports (Figs. 7-8) positioned distally forward of the inflatable balloon device 122. In the depicted embodiments, the distal ports of the tip element 129 comprise radial openings 127 facing in a generally radially outward direction and substantially uniformly spaced apart from one another along the circumference of the distal tip and further comprise one distal opening 128. As described in more detail below, the distal ports of the tip element 129 may all be in fluid communication with a single pressure sensor lumen 125 extending through the coronary sinus occlusion catheter 120. Accordingly, the coronary sinus pressure can be monitored via a pressure sensor device that is in fluid communication with the distal ports of the tip element 129.

Referring now to Fig. 2, the proximal portion 131 of the coronary sinus occlusion catheter 120 and the control system 140 are positioned external to the patient while the distal tip portion 121 is advanced into the coronary sinus 20. The proximal portion 131 includes the proximal hub 132 that is coupled to the control system 140 via a set of fluid or sensor lines 133, 134, and 135. As such, the control system 140 can activate or deactivate the occlusion component 122 at the distal tip portion 121 of the coronary sinus occlusion catheter 120 while also receiving one or more sensor signals that provide data indicative of heart characteristics (e.g., coronary sinus pressure, electrocardiogram (ECG) information, and the like).

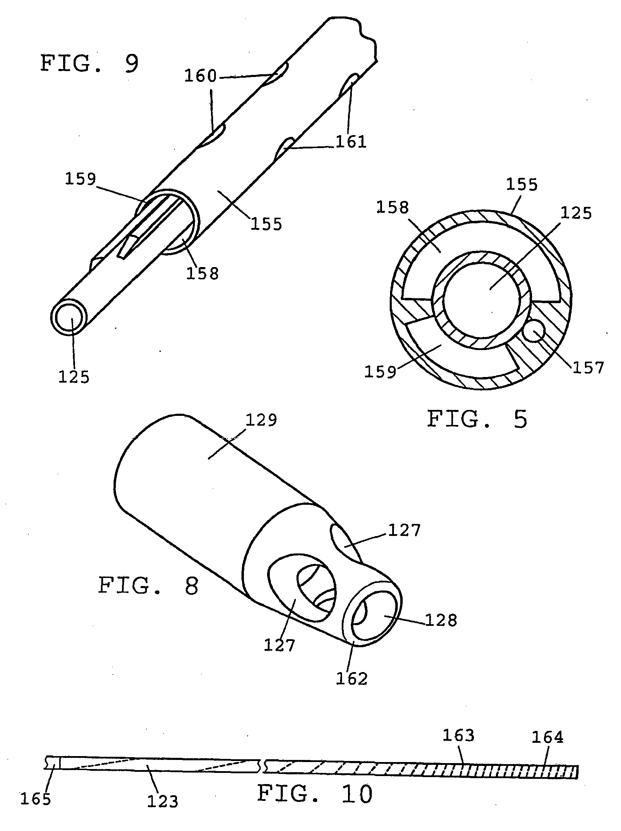

The proximal hub 132 of the coronary sinus occlusion catheter 120 serves to connect the plurality of fluid or sensor lines 133, 134, and 135 with the portion of the coronary sinus occlusion catheter 120 that extends into the patient's venous system. For example, the first line 133 extending between the control system 140 and the proximal hub 132 comprises a fluid line through which pressurized fluid (e.g., helium, another gas, or a stable liquid) can be delivered to activate the occlusion component (e.g., to inflate the inflatable balloon device 122). The fluid line 133 is connected to a corresponding port 143 of the control system 140 (e.g., the drive lumen port in this embodiment) so that the line 133 is in fluid communication with a pneumatic control subsystem housed in the control system 140. The proximal hub 132 joins the first line 133 with an inflation lumen 158 (Fig. 5) extending through the coronary sinus occlusion catheter 120 and to the inflatable balloon device 122.

In another example, the second line 134 extending between the control system 140 and the proximal hub 132 comprises a balloon sensor line that is in fluid communication with the interior of the inflatable balloon device 122 so as to measure the fluid pressure within the balloon device 122. The proximal hub 132 joins the second line 134 with a balloon pressure-monitoring lumen 159 (Fig. 5) extending through the coronary sinus occlusion catheter 120 and to the inflatable balloon device 122. The pressure of the balloon device 122 may be monitored an internal control circuit of the control system 140 as part of a safety feature that is employed to protect the coronary sinus 20 from an overly pressurized balloon device 122. The balloon sensor line 134 is connected to a corresponding port 144 of the control system 140 so that a pressure sensor arranged within the control system 140 can detect the fluid pressure in the balloon device 122. Alternatively, the pressure sensor may be arranged in the distal tip portion 121 (e.g., internal to the balloon device 122) or the in the proximal hub 132 such that only a sensor wire connects to the corresponding port 144 of the control system 140.

The proximal hub 132 also connects with a third line 135 extending from the control system 140. The third line 135 comprises a coronary sinus pressure line that is used to measure the fluid pressure in the coronary sinus both when the balloon device 122 is inflated and when it is deflated. The proximal hub 132 joins the third line 135 with a pressure sensor lumen 125 (Fig. 5) extending through the coronary sinus occlusion catheter 120 and to the distal openings 127, 128 of the catheter tip element 129 that are forward of the balloon device 122. As such, the pressure sensor lumen 125 can be a coronary sinus pressure lumen, with at least a portion of the third line 135 may operating as a fluid-filled path (e.g., saline, another biocompatible liquid, or a combination thereof) that transfers the blood pressure in the coronary sinus 20 to pressure sensor device 136 along a proximal portion of the third line 135. The pressure sensor device 136 samples the pressure measurements (which are indicative of the coronary sinus pressure) and outputs an sensor signal indicative of the coronary sinus pressure to the corresponding port 145 of the control system 140 for input to an internal control circuit (which may include one or more processors that execute instructions stored on one or more computer memory devices housed in the control system 140). The coronary sinus pressure data can be displayed by the graphical user interface 142 in a graph form so that a cardiologist or other user can readily monitor the trend of the coronary sinus pressure while the coronary sinus 20 is in an occluded condition and in a non-occluded condition. Optionally, the graphical user interface 142 of the control system 140 can also output a numeric pressure measurement on the screen so that the cardiologist can readily view a maximum coronary sinus pressure, a minimum coronary sinus pressure, or both. In alternative embodiments, the pressure sensor device 136 can be integrated into the housing of the control system 140 so that the third line 135 is a fluid-filled path leading up to the corresponding port 145, where the internal pressure sensor device (much like the device 136) samples the pressure measurements and outputs a signal indicative of the coronary sinus pressure.

Still referring to Fig. 2, the system 100 may include one or more ECG sensors 148 to output ECG signals to the control system 140. In this embodiment, the system 100 includes a pair of ECG sensor pads 148 that are adhered to the patient's skin proximate to the heart 10. The ECG sensors 148 are connected to the control system 140 via a cable that mates with a corresponding port 149 along the housing of the control system 140. As described in more detail below, the ECG data can be displayed by the graphical user interface 142 in a graph form so that a cardiologist or other user can readily monitor the patient's heart rate and other characteristics while the coronary sinus is in an occluded condition and in an non-occluded condition. Optionally, the graphical user interface 142 of the control system 140 can also output numeric heart rate data (based on the ECG sensor data on the screen so that the cardiologist can readily view the heart rate (e.g., in a unit of beats per minutes). The ECG sensor signals that are received by the control system 140 are also employed by the internal control circuit so as to properly time the start of the occlusion period (e.g., the start time at which the balloon device 122 is inflated) and the start of the non-occlusion period (e.g., the start time at which the balloon device 122 is deflated).

As shown in Fig. 2, the coronary sinus occlusion catheter 120 is delivered to the heart 10 via a venous system using any one of a number of venous access points. Such access points may be referred to as PICSO access points in some embodiments in which the coronary sinus occlusion catheter 120 is controlled to perform a PICSO procedure for at least a portion of the time in which the catheter 120 is positioned in the coronary sinus 20. For example, the guide member 110 and the distal tip portion 121 can be inserted into the venous system into an access point 12 at a brachial vein, an access point 13 at a subclavian vein, or at an access point 14 at a jugular vein. From any of these access points, the guide member 110 can be advanced through the superior vena cava and into the right atrium 11. Preferably, the guide member 110 is steered into an ostial portion of the coronary sinus 20, and then the distal tip portion 121 of the catheter 120 is slidably advanced along the guide member 110 and into the coronary sinus 20 before the guide member 110 is backed out to remain in the right atrium 11. In another example, the guide member 110 and the distal tip portion 121 can be inserted into the venous system into an access point 18 at a femoral vein. In this example, the guide member 110 can be advanced through the inferior vena cava and into the right atrium 11. As previously described, the distal tip portion 121 of the catheter 120 is slidably advanced along the guide member 110 and into the coronary sinus 20 before the guide member 110 is backed out to remain in the right atrium 11.

In some embodiments, the blockage 35 in the heart may be repaired or removed using a percutaneous coronary intervention (PCI) instrument such as an angioplasty balloon catheter, a stent delivery instrument, or the like. The PCI instrument may access the arterial system via any one of a number of PCI access points, as shown in Fig. 2. In some implementations, the PCI instrument can be inserted into the arterial system into an access point 15 at a femoral artery, an access point 16 at a radial artery, or an access point 17 at a subclavian artery. Thus, as previously described, some embodiments of the system 100 may employ a PICSO access point into the venous system while a PCI access point is employed to insert a PCI instrument into the arterial system.

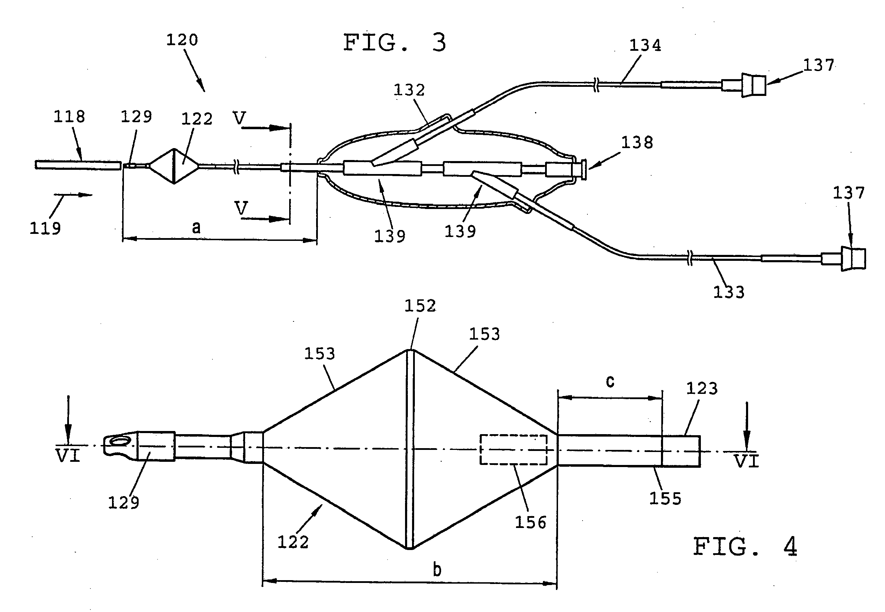

Referring now to Fig. 3, the catheter device 120 carries the balloon device 122 along its distal end portion. On its proximal end, the catheter 120 comprises a the hub portion 132, in which the proximal connection lines 133, 134, and 135 (not shown) are each connected with the respective lumens of the catheter 120 via a Y-connector 139. Alternatively, a multiple outlet for a plurality of feeds may be provided. One of the two proximal connection lines 133 is connected with the lumen (e.g., inflation lumen 158) serving to inflate and deflate the balloon 122 and carries a proximal connection piece 137 to which a fluid source may be connected. The other of the proximal connection lines 134 is connected with the lumen (balloon pressure-monitoring lumen 159) serving to measure the pressure internal to the balloon 122, and carries a proximal connection piece 137 which can be connected with an appropriate pressure measuring means. Finally, a Luer lock 138 may be guided out of the hub portion 132 for connection with the central lumen 125 (e.g., the pressure sensor lumen) of the catheter 120.

The distance between the tip of the catheter tip element 129 and the hub portion 132 is denoted by a and is, for instance, about one meter in order to enable the catheter to be introduced both via the jugular vein and via the femoral vein or the vein of the upper arm. A protective hose 118, which can be slipped over the catheter tip element 129 and the balloon 122 in the direction of arrow 119, is provided to protect the balloon 122 during the storage of the catheter 120.

From the detailed view according to Fig. 4, it is apparent that the balloon 122, in the inflated state, has a central, approximately cylindrical portion 152 which is adjoined by a conical portion 153 on either side, said conical portion 153 being each connected with the catheter shaft 155 via a collar-shaped end piece 154. In the inflated state, the diameter of the balloon 122 in the region of the cylindrical portion 152 may be, for instance, between about 12 mm and about 22 mm and, preferably, about 15 mm. The length b of the inflated portion of the balloon 122 may be, for instance, between about 20 mm and about 30 mm and, preferably, about 25 mm. A marker band 156 positioned in the balloon 122 can carry an X-ray-compatible material so as to be rendered visible during a surgery by suitable imaging processes.

In accordance with some embodiments, the catheter shaft 155 may include a plurality of lumens extending from the hub portion 132 to the distal portion 121 (e.g., to the balloon 122 or to the tip element 129). As shown in the cross-sectional illustration according to Fig. 5, the catheter 120 has the central lumen 125 (e.g., the pressure sensor lumen in this embodiment) as well as two ring segment-shaped lumens 158 and 159. The ring segment-shaped lumen 158 (e.g., the inflation lumen in this embodiment), which extends over a larger central angle than the ring segment-shaped lumen 159 (e.g., the balloon-pressure monitoring lumen in this embodiment), thereby providing a larger arc length than the ring segment-shaped lumen 159. The inflation lumen 158 likewise communicates with the interior of the balloon 122 and serves to inflate and deflate the balloon 122. The balloon-pressure monitoring lumen 159 likewise communicates with the interior of the balloon 122 and serves to measure the pressure prevailing within the balloon 122.

From Fig. 9 (which illustrates a portion of the catheter 120 that is interior to the balloon 122 (with the balloon 122 removed from view), it is apparent that the lumens 158 and 159 are each in communication with the interior of the balloon via two openings 160 and 161 respectively provided in an axially offset manner. The use of at least two radial openings 160 and 161 into the interior of the balloon 122 can reduce the risk of the balloon prematurely covering all openings, particularly during collapsing, i.e. prior to the complete evacuation of the balloon, so that further evacuation would be rendered difficult or impossible. Between the mutually adjacent ends of the ring segment-shaped lumens 158 and 159, a lumen 157 having a circular cross section is arranged. Through the lumen 157, electric wirings, sensors or the like can be conducted. Also, in some embodiments, in the lumen 157, a stiffening wire (refer to wire 165 in Fig. 7) can be arranged in the region of the balloon 122, as will be described in more detail below. In addition or in the alternative, electric wirings, sensors or the like can also be conducted through the central lumen 157.

Accordingly, the lumens 125, 157, 158, and 159 of the catheter device 120 may have cross-sectional geometries differing from one another. For example, the inflation lumen 158 that serves to inflate and/or deflate the balloon may have a ring segment-shaped in cross section and arranged radially outside the central lumen 125. The second lumen 159 (e.g., the balloon pressure-monitoring lumen in this embodiment) may have a ring segment-shaped in cross section and arranged radially outside the central lumen 125, and the arc-determining angle is preferably smaller as compared to the ring segment-shaped inflation lumen 158. Due to the fact that the ring segment-shaped cross section of the inflation lumen 158 extends over a larger central angle than the ring segment-shaped cross section of the balloon pressure-monitoring lumen 159, a larger cross section is provided for the inflation lumen 158 and, at the same time, separate pressure measurements will be enabled. The ability to provide pressure measurements via the separate lumen 159, for instance, has the advantage that possible buckling under flexural load (or kinking) can be detected. Buckling can be reliably detected due to the different pressures measured in the inflation lumen 158 and in the separate lumen 159. In such circumstances, it is thus feasible to take the respective safety measurements, e.g. actuate a safety valve, in a prompt manner. From the cross-sectional illustration according to Fig. 6, it is apparent that a stiffening element 123 in the form of a hypotube surrounds the catheter shaft 155 is arranged at a distance c (Fig. 4) from the proximal end of the balloon 2. An example embodiment of the hypotube 23 is illustrated in Fig. 10. As shown in Fig. 7, the stiffening wire 165 made, for instance, of nitinol may be arranged in the lumen 157. The stiffening wire 165 may extend over the length of the balloon 122 so as to reduce the likelihood of the catheter shaft 155 buckling or otherwise deforming in the region in which it is surrounded by the balloon 122, on account of the balloon pressure exerting axial upsetting forces on the catheter shaft 155.

An example embodiment of the catheter tip element 129 is illustrated in detail in Fig. 7. There, the attachment of the collar 154 of the balloon 122 to the distal end of the catheter is, in particular, illustrated. The connection is realized via an interposed, distal filling member 166, whereby a material adhesion of the collar 154 with the catheter upon interposition of the filling member 166 is effected in this region by thermal bonding or gluing. A further marker band 167 can be arranged at a distance d (e.g., about 6.5 mm in this embodiment) from the distal opening 128 of the catheter tip element 129. The catheter tip element 129 is connected with the distal end of the catheter by the aid of a transition piece 169. It is apparent that the axial lumen 126 of the catheter tip element 129 is arranged immediately consecutive to the central lumen 125 of the catheter. The catheter tip element 129 comprises a portion conically tapering toward the distal opening 128 and including three radial openings 127 uniformly distributed about its periphery.

From the perspective illustration according to Fig. 8, it is apparent that the edge 162 of the distal opening 128 is rounded off in order to avoid damage to the vessel inner wall.

Fig. 10 depicts a hypotube 123, which comprises a helical line-shaped notch 163. It is apparent that the pitch of the helix 163 in the distal end portion 164 is smaller than in a more proximally located region 165. This results in a reduced flexural strength in the distal end portion 164 so as to enable the catheter to better follow the various curvatures of the body vessel during its introduction. In some embodiments, the pitch of the helix 163 at the distal end portion 164 relative to the pitch of the helix at the proximally located region 165 can be selected so that the flexural strength is a function of the length of the catheter leading up to the balloon.

Thus, as described herein, the catheter 120 can be configured to provide sufficient rigidity in order to reduce the likelihood the occlusion catheter device will slip back from the occluded position because of the counter-pressure in the occluded vessel. At the same time, the catheter 120 may provide sufficient flexibility in order to enable the catheter 120 to be safely pushed forward through blood vessel regions having small radii of curvature so as to be able to position the inflatable balloon 122 on the catheter 120 at the desired site of application (e.g., the coronary sinus 20).

In some embodiments, the flexural strength of the balloon catheter 120 is obtained by the stiffening element 123 that surrounds the catheter shaft 155. The stiffening element 123 also facilitates the advancement of the balloon catheter. In order reduce the likelihood of injuring the vessel during the advancement of the catheter, and to permit advancement in curved regions having small radii of curvature, the distal end portion 164 of the stiffening element 123 can be positioned adjacent to the proximal end of the balloon 122 and may provide a flexural strength that is reduced relative to the remaining portion of the stiffening element. Thus, a region of higher flexibility is deliberately formed at the distal end portion 164 of the stiffening element 123 so as to enable the adaptation to a curved course of the body vessel during the advancement of the catheter 120, while preferably maintaining the balloon-carrying, distal portion 121 of the catheter 120 in a generally parallel relationship with the longitudinal extension of the respective vessel in order to avoid injury to the vessel wall.

In particular embodiments, the stiffening element 123 may extend substantially over the entire portion of the catheter shaft 155 between the balloon 122 and the proximal hub portion 132. In a preferred embodiment, the distance c is provided between the distal end of the stiffening element 123 and the proximal end of the inflatable region of the balloon 122. In the embodiment described herein, the distance c is dimensioned such that, on the one hand, the catheter shaft will not buckle between the distal end of the stiffening tube and the balloon, which would be the case with too large a distance, and, on the other hand, the flexibility and suppleness will not be limited too much in this region, which would be the case with too short a distance, or no distance at all. In some preferred embodiments, the distance c is about 4-6 mm and, in particular, about 5 mm.

As shown in Fig. 10, the stiffening element 123 may be formed by a separate stainless-steel tube or also by at least one outer layer co-extruded with the catheter shaft 155 and made of a synthetic material differing from that of the catheter shaft 155. Alternatively, the stiffening element 123 may be formed by a nylon tissue or comprise such a tissue. In some embodiments, the portion of the stiffening element having a reduced flexural strength extends over a length of about 30-120 mm, preferably about 40-90 mm, from the distal end of the stiffening element.

According to a preferred configuration, the hypotube 123 can include at least one notch influencing the flexural strength. In the embodiment depicted in Fig. 10, the notch 163 preferably extends in a helical line-shaped manner, with the helical line or helix having a smaller pitch in the portion of reduced flexural strength of the hypotube than in the remaining portion. As described herein, further optimization is feasible in that the pitch of the helix 163 continuously increases in the portion of reduced flexural strength of the hypotube, departing from the distal end of the hypotube. Due to the continuously variable flexural strength of the hypotube in the mentioned end portion, buckling sites will be avoided.

In alternative embodiments, instead of a helical line-shaped notch 163, a plurality of notches offset in the axial direction may also be provided on the stiffening element. Each of the notches can extend over a partial circumference of the hypotube, with the flexural strength depending on the axial distance between the individual notches. A number of embodiments of the invention have been described. Nevertheless, it will be understood that various modifications may be made without departing from the scope of the invention. Accordingly, other embodiments are within the scope of the following claims.

## Claims

1. A balloon catheter for introduction into a body vessel, in particular the coronary sinus, and for intermittently occluding the same, comprising a catheter shaft (155) that carries an inflatable balloon (122) on a distal end portion and a plurality of lumens extending through the catheter shaft (155), that a pressure sensor lumen (125) extending centrally through the catheter shaft (155) to at least one distal opening (127,128) is provided, the at least one distal opening (127, 128) being arranged distally of the balloon (122) for communication with the fluid in the body vessel, and that, furthermore, an inflation lumen (158) to inflate and deflate the balloon (122) is provided, and that the catheter comprises a stiffening element (123) extending along the catheter shaft (155), the stiffening element (123) having a distal end portion (164) that is positioned adjacent to the proximal end of the balloon (122) and has a flexural strength reduced relative to a remaining portion of the stiffening element (123), **characterized in that** a balloon pressure-monitoring lumen (159) to measure the pressure in the balloon (122) is further provided.

2. A balloon catheter according to claim 1, **characterized in that** the stiffening element comprises a hypotube (123) surrounding the catheter shaft (155).

3. A balloon catheter according to claim 1 or 2, **characterized in that** the stiffening element (123) substantially extends over the entire portion of the catheter shaft (155) between the balloon (122) and a proximal hub (132).

4. A balloon catheter according to claim 1, 2 or 3, **characterized in that** the distal end portion (164) of the stiffening element (123) having a reduced flexural strength extends over a length of 30-120 mm, preferably 40-90 mm.

5. A balloon catheter according to claim 2, 3 or 4, **characterized in that** the hypotube (123) comprises at least one notch (163) influencing the flexural strength.

6. A balloon catheter according to claim 5, **characterized in that** the notch (163) extends in a helical line-shaped manner, with the helical line having a smaller pitch in the distal end portion (164) of the stiffening element (123) having the reduced flexural strength than in the remaining portion of the stiffening element (123).

7. A balloon catheter according to claim 6, **characterized in that** the pitch of the helical line continuously increases in the distal end portion (164) of the stiffening element (123) having the reduced flexural strength, departing from the distal end of the stiffening element (123).

8. A balloon catheter according to any one of claims 1 to 7, **characterized in that** the lumens (125,157,158,159) of the catheter have cross-sectional geometries differing from one another.

9. A balloon catheter according to any one of claims 1 to 8, **characterized in that** the inflating lumen (158) has is ring segment-shaped in cross section and is arranged radially outside the pressure sensor lumen (125).

10. A balloon catheter according to claim 9, **characterized in that** the balloon pressure-monitoring lumen (159) is ring segment-shaped in cross section and arranged radially outside the pressure sensor lumen (125), wherein the arc-determining angle of the balloon pressure-monitoring lumen (159) is preferably smaller as compared to the ring segment-shaped inflation lumen (158) .

11. A balloon catheter according to claim 10, **characterized in that** a circular or oval lumen (157) is provided between the neighbouring ends of the ring segment-shaped cross sections of the inflation lumen (158) and the balloon pressure-monitoring lumen (159).

12. A balloon catheter according to any one of claims 1 to 10, **characterized in that** the inflation lumen (158) and optionally the balloon pressure-monitoring lumen (159) are each connected with the interior of the balloon (122) via at least two radial openings (160,161) arranged to be offset in the axial direction of the catheter shaft (155).

13. A balloon catheter according to any one of claims 1 to 12, **characterized in that** the catheter comprises a stiffening means, in particular a stiffening wire (165), in the region of the balloon (122), said wire (165) preferably extending over the length of the balloon (122).

14. A balloon catheter according to any one of claims 1 to 13, further comprising a catheter tip element (129) in fluid communication with the pressure sensor lumen (125) extending centrally through the catheter shaft (155), the catheter tip element (129) comprising a plurality of distal ports that are positioned distally of the balloon (122) for communication with fluid in the body vessel, the plurality of distal ports comprising a plurality of radial openings (127), preferably at least two, in particular three radial openings (127), that are substantially uniformly distributed over a circumferential periphery of the catheter tip element (129).

15. A balloon catheter according to claim 14, **characterized in that** a distal edge of a distal opening (128) of the catheter tip element (129) is rounded off.

16. A balloon catheter according to claim 14 or 15, **characterized in that** the catheter tip element (129) conically tapers toward the distal opening (128).

17. A balloon catheter according to claim 14, 15 or 16, **characterized in that** the catheter tip element (129) is flexible.

18. A balloon catheter according to any one of claims 14 to 17, **characterized in that** the distance between the distal end of the catheter tip element (129) and the distal end of the inflatable portion of the balloon (122) is at least 30 mm and, preferably, 35 to 45 mm.

## Patentansprüche

1. Ballonkatheter zum Einführen in ein Hohlorgan, insbesondere den Koronarsinus, und das Okkludieren desselben umfassend einen Katheterschaft (155), der an seinem distalen Endbereich einen aufblasbaren Ballon (122) trägt, und eine Mehrzahl von sich durch den Katherterschaft erstreckenden Lumina, wobei ein der Druckmessung dienendes Lumen (125) vorgesehen ist, das sich zentral durch den Katheterschaft (155) bis zu wenigstens einer distalen Öffnung (127, 128) erstreckt, dessen wenigstens eine distale Öffnung (127, 128) distal des Ballons (122) angeordnet ist, um mit der Flüssigkeit im Hohlorgan zu kommunizieren, und weiters ein der Befüllung und der Entleerung des Ballons (122) dienendes und mit diesem in Verbindung stehendes Lumen (158) vorgesehen ist, und wobei der Katheter ein sich entlang des Katheterschaftes (155) erstreckendes Versteifungselement (123) aufweist, dessen distaler, dem proximalen Ende des Ballons (122) benachbarter Endbereich (164) eine gegenüber einem verbleibenden Bereich des Versteifungselements (123) geringere Biegesteifigkeit aufweist, **dadurch gekennzeichnet, dass** weiters ein der Druckmessung des Ballons dienendes Lumen (159) vorgesehen ist, um den Druck im Ballon (122) zu messen.

2. Ballonkatheter nach Anspruch 1, **dadurch gekennzeichnet, dass** das Versteifungselement von einem den Katheterschaft (155) umgebenden Kanülenrohr (123) gebildet ist.

3. Ballonkatheter nach Anspruch 1 oder 2, **dadurch gekennzeichnet, dass** sich das Versteifungselement (123) im Wesentlichen über den gesamten Abschnitt des Katheterschaftes (155) zwischen dem Ballon (122) und einem proximalen Einspeisteil (132) erstreckt.

4. Ballonkatheter nach Anspruch 1, 2 oder 3, **dadurch gekennzeichnet, dass** der distale Endbereich (164) mit geringerer Biegesteifigkeit des Versteifungselements (123) sich über eine Länge von 30-120 mm, vorzugsweise 40-90 mm erstreckt.

5. Ballonkatheter nach Anspruch 2, 3 oder 4, **dadurch gekennzeichnet, dass** das Kanülenrohr (123) wenigstens einen die Biegesteifigkeit beeinflussenden Einschnitt (163) trägt.

6. Ballonkatheter nach Anspruch 5, **dadurch gekennzeichnet, dass** der Einschnitt (163) schraubenlinienförmig verläuft, wobei die Schraubenlinie im distalen Endbereich (164) des Versteifungselements (123) mit geringerer Biegesteifigkeit (34) eine geringere Steigung aufweist als in dem übrigen Bereich des Versteifungselements (123).

7. Ballonkatheter nach Anspruch 6, **dadurch gekennzeichnet, dass** die Steigung der Schraubenlinie im Bereich der geringeren Beigesteifigkeit des Versteifungslements (123) vom distalen Ende des Versteifungselements (123) weg kontinuierlich zunimmt.

8. Ballonkatheter nach einem der Ansprüche 1 bis 7, **dadurch gekennzeichnet, dass** die Lumen (125, 157, 158, 159) des Katheters eine voneinander verschiedene Querschnittsgeometrie aufweisen.

9. Ballonkatheter nach einem der Ansprüche 1 bis 8, **dadurch gekennzeichnet, dass** das der Befüllung und der Entleerung des Ballons dienende Lumen (158) im Querschnitt ringsegmentförmig ausgebildet und radial außerhalb des der Druckmessung dienenden Lumens (125) angeordnet ist.

10. Ballonkatheter nach Anspruch 9, **dadurch gekennzeichnet, dass** das der Druckmessung im Ballon dienende Lumen (159) im Querschnitt ringsegmentförmig ausgebildet und radial außerhalb des der Druckmessung dienenden Lumens (125) angeordnet ist, wobei der den Segmentbogen bestimmende Winkel des der Druckmessung dienenden Lumens (159) im Vergleich zum der Befüllung und der Entleerung des Ballons dienenden ringsegmentförmigen Lumen (158) bevorzugt geringer ist.

11. Ballonkatheter nach Anspruch 10, **dadurch gekennzeichnet, dass** ein kreisförmiges oder ovales Lumen (157) zwischen den einander benachbarten Enden der ringsegmentförmigen Abschnitte der der Befüllung und der Entleerung des Ballons dienenden Lumina (158) und dem der Druckmessung dienenden Lumen (159) angeordnet ist.

12. Ballonkatheter nach einem der Ansprüche 1 bis 10, **dadurch gekennzeichnet, dass** das der Befüllung und der Entleerung des Ballons dienende Lumen (158) und ggf. das der Druckmessung im Ballon dienende Lumen (159) über jeweils wenigstens zwei in axialer Richtung des Katheterschafts (155) versetzt angeordnete radiale Durchbrechungen (160, 161) mit dem Inneren des Ballons (122) verbunden sind.

13. Ballonkatheter nach einem der Ansprüche 1 bis 12, **dadurch gekennzeichnet, dass** der Katheter im Bereich des Ballons (122) ein Versteifungsmittel, insbesondere einen Versteifungsdraht (165) aufweist, der sich bevorzugt über die Länge des Ballons (122) erstreckt.

14. Ballonkatheter nach einem der Ansprüche 1 bis 13, weiters enthaltend ein Katheterspitzenelement (129), das in Fluidkommunikation mit dem der Druckmessung dienenden Lumen (125), das sich zentral durch den Katheterschaft (155) erstreckt, steht, wobei das Katheterspitzenelement (129) eine Mehrzahl von distalen Öffnungen, die distal vom Ballon platziert sind, besitzt, um mit der Flüssigkeit im Hohlorgan zu kommunizieren, wobei die Mehrzahl an distalen Öffnungen eine Mehrzahl radialer Durchbrechungen (127), bevorzugt wenigstens zwei, bevorzugt drei über den Umfang des Katheterspitzenelements (129) gleichmäßig verteilte radiale Durchbrechungen (127) aufweist.

15. Ballonkatheter nach Anspruch 14, **dadurch gekennzeichnet, dass** der Rand der distalen Öffnung (128) des Katheterspitzenelements (129) abgerundet ausgebildet ist.

16. Ballonkatheter nach Anspruch 14 oder 15, **dadurch gekennzeichnet, dass** das Katheterspitzenelement (129) zur distalen Öffnung (128) hin konisch zuläuft.

17. Ballonkatheter nach Anspruch 14, 15 oder 16, **dadurch gekennzeichnet, dass** das Katheterspitzenelement (129) biegsam ist.

18. Ballonkatheter nach einem der Ansprüche 14 bis 17, **dadurch gekennzeichnet, dass** der Abstand zwischen der distalen Spitze des Katheterspitzenelements (129) und dem distalen Ende des aufblasbaren Bereich des Ballons (122) wenigstens 30mm, bevorzugt 35 bis 45mm beträgt.

## Revendications

1. Cathéter à ballonnet à introduire dans un vaisseau corporel, en particulier le sinus coronaire, et visant à l'obstruer par intermittence, comprenant un arbre de cathéter (155) qui porte un ballonnet gonflable (122) sur une partie d'extrémité distale et une pluralité de lumières s'étendant à travers l'arbre de cathéter (155), dans lequel une lumière pour capteur de pression (125) s'étendant de manière centrale à travers l'arbre de cathéter (155) jusqu'à au moins une ouverture distale (127, 128) est prévue, ladite au moins une ouverture distale (127, 128) étant disposée de manière distale par rapport au ballonnet (122) pour permettre la communication avec le fluide dans le vaisseau corporel, et dans lequel en outre une lumière de gonflage (158), permettant de gonfler et de dégonfler le ballonnet (122) est prévue, et dans lequel le cathéter comprend un élément de renfort (123) s'étendant le long de l'arbre de cathéter (155), l'élément de renfort (123) ayant une partie d'extrémité distale (164) qui est positionnée de manière adjacente à l'extrémité proximale du ballonnet (122) et a une résistance à la flexion réduite par rapport à une partie restante de l'élément de renfort (123), **caractérisé en ce qu'**une lumière de contrôle de la pression du ballonnet (159), pour mesurer la pression dans le ballonnet (122), est en outre prévue.

2. Cathéter à ballonnet selon la revendication 1, **caractérisé en ce que** l'élément de renfort comprend un hypotube (123) entourant l'arbre de cathéter (155).

3. Cathéter à ballonnet selon la revendication 1 ou 2, **caractérisé en ce que** l'élément de renfort (123) s'étend essentiellement sur toute la partie de l'arbre de cathéter (155) entre le ballonnet (122) et un moyeu proximal (132).

4. Cathéter à ballonnet selon les revendications 1, 2 ou 3, **caractérisé en ce que** la partie d'extrémité distale (164) de l'élément de renfort (123) ayant une résistance à la flexion réduite s'étend sur une longueur de 30-120 mm, de préférence 40-90 mm.

5. Cathéter à ballonnet selon les revendications 2, 3 ou 4, **caractérisé en ce que** l'hypotube (123) comprend au moins une encoche (163) influençant la résistance à la flexion.

6. Cathéter à ballonnet selon la revendication 5, **caractérisé en ce que** l'encoche (163) s'étend en forme de ligne hélicoïdale, la ligne hélicoïdale ayant un pas plus petit dans la partie d'extrémité distale (164) de l'élément de renfort (123) dont la résistance à la flexion est réduite, que dans la partie restante de l'élément de renfort (123).

7. Cathéter à ballonnet selon la revendication 6, **caractérisé en ce que** le pas de la ligne hélicoïdale augmente continuellement dans la partie d'extrémité distale (164) de l'élément de renfort (123) dont la résistance à la flexion est réduite, en partant de l'extrémité distale de l'élément de renfort (123).

8. Cathéter à ballonnet selon l'une quelconque des revendications 1 à 7, **caractérisé en ce que** les lumières (125, 157, 158, 159) du cathéter ont des géométries transversales différentes les unes des autres.

9. Cathéter à ballonnet selon l'une quelconque des revendications 1 à 8, **caractérisé en ce que** la lumière de gonflage (158) a une forme de segment annulaire en coupe transversale et est disposée radialement hors de la lumière du capteur de pression (125).

10. Cathéter à ballonnet selon la revendication 9, **caractérisé en ce que** la lumière de contrôle de la pression du ballonnet (159) est en forme de segment annulaire en coupe transversale et disposée radialement hors de la lumière de capteur de pression (125), dans lequel l'angle de détermination de l'arc de la lumière de contrôle de la pression du ballonnet (159) est de préférence inférieur à la lumière de gonflage en forme de segment annulaire (158).

11. Cathéter à ballonnet selon la revendication 10, **caractérisé en ce qu'**une lumière circulaire ou ovale (157) est prévue entre les extrémités voisines des sections transversales en forme de segment annulaire de la lumière de gonflage (158) et de la lumière de contrôle de la pression du ballonnet (159).

12. Cathéter à ballonnet selon l'une quelconque des revendications 1 à 10, **caractérisé en ce que** la lumière de gonflage (158) et éventuellement la lumière de contrôle de la pression du ballonnet (159) sont chacune raccordées à l'intérieur du ballonnet (122) par le biais d'au moins deux ouvertures radiales (160, 161) disposées, de manière à être décalées dans la direction axiale de l'arbre de cathéter (155).

13. Cathéter à ballonnet selon l'une quelconque des revendications 1 à 12, **caractérisé en ce que** le cathéter comprend un moyen de renfort, en particulier un fil de renfort (165), dans la région du ballonnet (122), ledit fil (165) s'étendant de préférence sur toute la longueur du ballonnet (122).

14. Cathéter à ballonnet selon l'une quelconque des revendications 1 à 13, comprenant en outre un élément de pointe de cathéter (129) en communication fluidique avec la lumière du capteur de pression (125) s'étendant de manière centrale à travers l'arbre de cathéter (155), ledit élément de pointe de cathéter (129) comprenant une pluralité d'orifices distaux qui sont positionnés de manière distale par rapport au ballonnet (122), de façon à communiquer avec le fluide dans le vaisseau corporel, la pluralité d'orifices distaux comprenant une pluralité d'ouvertures radiales (127), de préférence au moins deux, en particulier trois ouvertures radiales (127), qui sont réparties de manière sensiblement uniforme sur une périphérie circonférentielle de l'élément de pointe de cathéter (129).

15. Cathéter à ballonnet selon la revendication 14, **caractérisé en ce qu'**un bord distal d'une ouverture distale (128) de l'élément de pointe de cathéter (129) est arrondi.

16. Cathéter à ballonnet selon la revendication 14 ou 15, **caractérisé en ce que** l'élément de pointe de cathéter (129) se rétrécit de manière conique vers l'extrémité distale (128).

17. Cathéter à ballonnet selon la revendication 14, 15 ou 16, **caractérisé en ce que** l'élément de pointe de cathéter (129) est flexible.

18. Cathéter à ballonnet selon l'une quelconque des revendications 14 à 17, **caractérisé en ce que** la distance entre l'extrémité distale de l'élément de pointe de cathéter (129) et l'extrémité distale de la partie gonflable du ballonnet (122) est d'au moins 30 mm et, de préférence de 35 à 45 mm.
